# EUROPEAN PATENT SPECIFICATION

(11) **EP 2 182 940 B1**
(45) Date of publication and mention of the grant of the patent: **12.03.2014**
(21) Application number: 08762489.6
(22) Date of filing: 25.06.2008
(51) Int. Cl.: A61K 31/352, A61K 36/185, A61P 37/00

(54) **USE OF TETRAHYDROCANNABINOL AND/OR CANNABIDIOL FOR THE TREATMENT OF INFLAMMATORY BOWEL DISEASE**
VERWENDUNG VON TETRAHYDROCANNABINOL UND/ODER CANNABIDIOL ZUR BEHANDLUNG VON ENTZÜNDLICHER DARMERKRANKUNG
UTILISATION DE TÉTRAHYDROCANNABINOL ET/OU DU CANNABIDIOL POUR LE TRAITEMENT D'UNE MALADIE INFLAMMATOIRE DE L'INTESTIN

(30) Priority: 05.07.2007 GB 0713083
(43) Date of publication of application: 12.05.2010
(73) Proprietor: GW Pharma Limited, Salisbury Wiltshire SP4 0JR (GB)
(72) Inventor: ROBSON, Philip, Salisbury, Wiltshire SP4 0JQ (GB); GUY, Geoffrey, Salisbury, Wiltshire SP4 0JQ (GB); PERTWEE, Roger, Aberdeen, Aberdeenshire AB25 2ZD (GB); JAMONTT, Joanna, Hatfield, Hartfordshire AL10 9AB (GB)
(74) Representative: Wells, Andrew
(86) International application number: PCT/GB2008/002183
(87) International publication number: WO 2009/004302

(56) References cited:
- WO-A-99/52524
- WO-A-2006/024958
- GB-A- 2 377 633
- US-A1- 2004 138 293
- FRIDE E ET AL: "Peripheral, but not central effects of cannabidiol derivatives: Mediation by CB1 and unidentified receptors" NEUROPHARMACOLOGY, PERGAMON PRESS, OXFORD, GB, vol. 48, no. 8, 1 June 2005 (2005-06-01), pages 1117-1129, XP004907368 ISSN: 0028-3908
- KLEIN THOMAS W ET AL: "Therapeutic potential of cannabinoid-based drugs." ADVANCES IN EXPERIMENTAL MEDICINE AND BIOLOGY 2007, vol. 601, 2007, pages 395-413, XP009107026 ISSN: 0065-2598

## Description

### FIELD OF THE INVENTION

The present invention relates to the use of a combination of the cannabinoids tetrahydrocannabinol (THC) and cannabidiol (CBD) in the treatment of an inflammatory bowel disease, wherein the ratio of THC to CBD (w/w) is 2:1 to 1:2, and wherein the CBD is in a unit dose form suitable for delivering a daily dose of CBD in the dose range of from between 5 to 200mg.

### BACKGROUND DESCRIPTION

Inflammatory bowel disease is the term used to describe chronic diseases that cause inflammation of the intestines. Diseases such as ulcerative colitis and Crohn's disease are examples of inflammatory bowel conditions.

Ulcerative colitis is an inflammatory disease of the colon. In ulcerative colitis, the lining of the intestinal wall reddens and swells and develops ulcers. The condition is often most severe in the rectal area, which can cause frequent diarrhoea. Additionally mucus and blood may appear in the stool if the lining of the colon is damaged.

Crohn's disease may affect any part of the digestive system, from the mouth to the anus, but it is most common in the lower part of the small bowel or the first part of the large bowel. It often affects more than one part of the bowel leaving normal, unaffected areas in between.

Crohn's disease is a chronic relapsing inflammatory disorder of the gastrointestinal (GI) tract occurring worldwide, most notably in North America and Europe with an incidence of 2-6 per 100,000 per year and a prevalence of 60-80 per 100,000. The onset of disease can occur in any age group but is more common in young adults. Crohn's disease is characterised by mucosal membrane inflammation affecting any part of the GI tract, with the terminal ileum (35%), ileocaecal region (40%) and colon (20%) generally being the most affected areas.

Crohn's disease patients have an increased blood flow in the wall of the bowel; this causes inflammation and ulceration, which extends to the deepest layers of the bowel.

The exact cause of Crohn's disease is not known, but it is thought that the body's immune system overreacts to a virus or bacterium, causing ongoing inflammation in the bowel. The disease often tends to run in families.

Crohn's disease is usually a life-long condition, with alternating flare-ups of symptoms and periods of remission. The symptoms include: diarrhoea, up to 10 or 20 times a day; pain, anywhere in the abdomen, and is often described as cramping or colicky. The abdomen may be sore to the touch and swollen; loss of appetite; weight loss; fever; rectal bleeding; anaemia; fissures and abscesses in the anal area.

During a flare-up of symptoms problems in other areas of the body may also occur, such as mouth ulcers, joint pain, eye inflammation, rashes and ulcers on the skin.

With chronic Crohn's disease, severe inflammation may cause complications to develop. This includes a fistula, which is an abnormal connection between the bowel and a neighbouring part of the body, such as the bladder, vagina, or another loop of bowel. Fistulas may lead to recurrent infections of the urinary or genital tracts. Other complications include an abscess (collection of pus) inside the abdomen or a stricture, a narrowing of the bowel caused by scar tissue that can obstruct the passage of material through the bowel. It is also known that patients who have had Crohn's disease for 8 to 10 years are at an increased risk of bowel cancer.

There is no cure for Crohn's disease. Symptoms can be improved with dietary changes, drugs or surgery, or a combination of these.

Medicines to reduce inflammation such as corticosteroids are often used in Crohn's disease along with medicines that suppress the immune system. Anti-diarrhoea medicines, antibiotics and painkillers may also be used during flare-ups.

Existing therapies currently available for use in the treatment of acute active Crohn's disease, particularly corticosteroids, are not universally effective or well tolerated by all patients and/or may not be cost-effective in the long-term. In addition, approximately 45% of patients cannot discontinue corticosteroid treatment without an exacerbation of their disease and consequently, many patients can become tolerant to such drugs although, this is more evident in moderate-severe disease.

Many people with Crohn's disease require surgical treatment at some time to treat complications such as anal abscesses, or fistulae, to remove areas of narrowed, non-functioning bowel, or when drugs are not controlling the disease.

About 15-20% of patients have frequent flare-ups, whereas a few have only one or two bouts of Crohn's disease during their lifetime. Most people are somewhere in between and are able to continue with normal life activities once Crohn's disease has been diagnosed and treatment has started. Unfortunately these are unpredictable.

The use of cannabis as a medicine has long been known and during the 19^{th} Century, preparations of cannabis were recommended as a hypnotic sedative which were useful for the treatment of hysteria, delirium, epilepsy, nervous insomnia, migraine, pain and dysmenorrhoea.

Until recent times the administration of cannabis to a patient could only be achieved by preparation of cannabis by decoction which could then be swallowed, or by the patient inhaling the vapours of cannabis by smoking the dried plant material. Recent methods have sought to find new ways to deliver cannabinoids to a patient including those which bypass the stomach and the associated first pass effect of the liver which can remove up to 90% of the active ingested dose and avoid the patient having to inhale unhealthy tars and associated carcinogens into their lungs.

Formulations containing specific, defined ratios of cannabinoids may be formulated from pure, synthetic cannabinoids or from extracts derived from the cannabis plant in combination with pharmaceutical carriers and excipients or combinations thereof.

Cannabinoids are a group of chemicals known to activate cannabinoid receptors in cells. These chemicals, which are found in cannabis plants, are also produced endogenously in humans and other animals, these are termed endocannabinoids. Synthetic cannabinoids are chemicals with similar structures to plant cannabinoids or endocannabinoids.

To date there has been no clinical research to assess the use of cannabinoids in the treatment of inflammatory bowel disease. Anecdotal reports suggest that patients suffering from Crohn's disease using whole cannabis either as a decoction or by smoking can obtain relief of symptoms.

The applicant's co-pending patent applications (EP 1361864 and EP1542657) suggests that the use of a broad ratio CBD product might be useful in the treatment of inflammatory bowel disease, although it fails to provide any data to the potential usefulness of such a product.

Furthermore the European patent EP1071417 (Feldmann et al.) describes the use of pure cannabidiol. The pure CBD is thought to be useful as an anti-inflammatory agent and much data is provided for its use as a treatment for rheumatoid arthritis. However, there is no data to support its use in the treatment of inflammatory bowel disease.

In addition there are many published reports (Fride et al. (2005) and Di Carlo and Izzo (2003)) which describe the usefulness of CBD as an anti-inflammatory and as such making CBD a useful treatment in inflammatory bowel disease.

Also Massa and Monory in 2006 describes the use of the bodies natural cannabinoids, endocannabinoids as natural protectants in inflammatory and gastrointestinal disorders.

Although there is data relating to the use of cannabidiol in inflammatory bowel disease there has been no progress in production of a medicament that can be used by a patient suffering from inflammatory bowel disease. This may be due to the data generated in *in vitro* and *in vivo* animal models not transpiring an effect in clinical patients actually suffering from the disease.

As is clear from the above there is an unmet requirement for an efficacious treatment for inflammatory bowel disease and it is thought that the administration of cannabinoids alone and in combination with each other or as an adjunct medicament may be efficacious in the treatment of patients suffering from inflammatory bowel disease.

The present invention describes how both the cannabinoids cannabidiol (CBD) and tetrahydrocannabinol (THC) are useful in the treatment of inflammatory bowel disease, either alone or more particularly when used in combination.

Additionally it was found that the use of CBD was particularly useful as an adjunct therapy with other medications used in the treatment of inflammatory bowel disease. In particular the other medicaments may include one or more of the following: aminosalicylates; corticosteroids; and immunosupressants. This data has been generated in patients actually suffering from inflammatory bowel disease and as such gives credence to the data.

### SUMMARY OF THE INVENTION

According to the first aspect of the present invention there is provided a combination of the cannabinoids tetrahydrocannabinol (THC) and cannabidiol (CBD) for use in the treatment of an inflammatory bowel disease, wherein the ratio of THC to CBD (w/w) is 2:1 to 1:2, and wherein the CBD is in a unit dose form suitable for delivering a daily dose of CBD in the dose range of from between 5 to 200mg.

More preferably still the THC and CBD may be administered separately, sequentially or simultaneously to one another.

A plant extract is defined as an extract from a plant material as described by the Guidance for Industry Botanical Drug Products Draft Guidance, August 2000, US Department of Health and Human Services, Food and Drug Administration Centre for Drug Evaluation and Research.

Plant material is defined as a plant or plant part (e.g. bark, wood, leaves, stems, roots, flowers, fruits, seeds, berries or parts thereof) as well as exudates.

Botanical drug substances which are derived from cannabis plants include primary extracts prepared by such processes as for example, maceration, percolation, extraction with solvents such as C1 to C5 alcohols (e.g. ethanol), Norflurane (HFA134a), HFA227, liquid carbon dioxide under pressure and extraction using a hot gas.

A primary extract may be further purified by supercritical or subcritical extraction, vaporisation and chromatography. When solvents such as those listed above are used the resultant extract may contain non-specific lipid-soluble material. This can be removed by a variety of processes including winterisation, which involves chilling to -20°C followed by filtration to remove waxy ballast, extraction with liquid carbon dioxide and by distillation.

Botanical drug substances are formulated into Botanical Drug Products which are defined in the Guidance for Industry Botanical Drug Products Draft Guidance, August 2000, US Department of Health and Human Services, Food and Drug Administration Centre for Drug Evaluation and Research as: "A botanical product that is intended for use as a drug; a drug product that is prepared from a botanical drug substance."

The term "inflammatory bowel disease" is used to describe diseases associated with inflammation of the bowel. These include but are not limited to: Crohn's disease and ulcerative colitis.

Preferably the inflammatory bowel disease is Crohn's disease.

Alternatively the inflammatory bowel disease is ulcerative colitis.

Certain aspects of this invention are further described, by way of example only.

### SPECIFIC DESCRIPTION

Recently, clinical trials have been performed on cannabinoids, in order to test the mainly anecdotal evidence of their analgesic and other medicinal properties.

One study has found that the combination of tetrahydrocannabinol (THC) and cannabidiol (CBD) in an approximately equal ratio was an effective analgesic in patients with central neuropathic pain (Berman et al., 2004). The cannabinoid-containing plant extracts of *Cannabis Sativa L.* containing either THC or CBD were mixed in a 1:1 ratio and compared against placebo.

It has been suggested that there may be an interaction between the cannabinoid components in a cannabis plant extract with other non-cannabinoid components in the plant extract.

The components of the THC and CBD plant extracts used in the following examples are described in Table 1 below.

**Table 1: components of the THC and CBD plant extracts**

| | THC-rich extract (% w/w of extract) | CBD-rich extract (% w/w of extract) |
|---|---|---|
| Primary/Secondary Cannabinoid: | | |
| THC Content | 63.0 - 78.0 | 2.0 - 6.5 |
| CBD Content | 0.1 - 1.5 | 57.0 - 72.0 |

| Other Cannabinoids: | | |
|---|---|---|
| Cannabigerol | 1.0 - 2.0 | 0.8 - 6.5 |
| Cannabichromene | 0.8 - 2.2 | 3.0 - 6.5 |
| Tetrahyrocannabid-ivarin | 0.4 - 1.0 | - |
| Tetrahydrocannabin-olic acid | <2.0 | - |
| Cannabidivarin | - | 1.0 - 2.0 |
| Cannabidiolic acid | - | <2.0 |

| Terpenes: | | |
|---|---|---|
| Monoterpenes | 0.7 | 0.4 |
| Di/tri-terpenes | 0.6 | 0.4 |
| Sesquiterpenes | 1.7 | 2.0 |

The "primary cannabinoid" is herein defined as the predominant cannabinoid in a single cannabinoid-containing plant extract. In the case of a plant extract from a cannabis plant bred to contain a high content of THC the primary cannabinoid will be THC.

The "secondary cannabinoid" is herein defined as the second most predominant cannabinoid in a single cannabinoid-containing plant extract. In the case of a plant extract from a cannabis plant bred to contain a high content of THC the secondary cannabinoid will usually be CBD.

The "other cannabinoids" are herein defined as all of the remaining cannabinoids that are present in a cannabis plant extract when the primary and the secondary cannabinoids have been accounted for. In the case of a plant extract from a cannabis plant bred to contain a high content of CBD the other cannabinoids will include cannabigerol (CBG), cannabichromene (CBC), cannabidivarin (CBDV) and cannabidiolic acid (CBDA).

It should be noted that in a cannabis plant all of the cannabinoids will naturally occur as their acid form. This form will decarboxylate to its neutral form over time once extracted. For the purpose of this document, when reference is made to a cannabinoid either the neutral or acid form are intended.

The non-cannabinoid containing fraction will usually comprise terpenes; which usually account for approximately 6% (w/w) of the total weight of the extract and other plant derived components, which account for 1-28% (w/w) of the total weight of the extract. The other plant derived components include sterols, triglycerides, alkanes, squalene, tocopherol and carotenoids.

The above ranges and compounds are from analysis of a cannabinoid-containing plant extract which was extracted from a cannabis plant using the subcritical CO₂ extraction technique as described in the applicants granted United Kingdom patent GB2391865.

The International patent application WO 2002/32420 in the name of Delta-9-Pharma describes in Table 1 the composition of cannabis plant extracts that have been extracted using other techniques. Other components of the non-cannabinoid containing fraction have been identified using supercritical CO₂ extraction, ethanol and hexane extraction techniques. These include: chlorophyll, flavinoids, glycosides and alkaloids.

Another cannabis plant extraction technique is extraction with hot gas as described in the applicants granted United Kingdom patent GB2376464.

### Example 1

Cannabidiol (CBD) has been shown to interact with tetrahydrocannabinol (THC) in behavioural studies, but it is not known if these cannabinoids interact in vivo in inflammatory disorders.

In this example, the TNBS (trinitrobenzenesulfonic acid) model of colitis was used to characterize the effects of CBD alone, THC alone and a combination of CBD and THC (in two different ratios) on inflammation and *in vitro* motility and secretory parameters.

Colitis was evoked in male Wistar rats by intra-colonic administration of 6.7mg TNBS in 0.25ml 25% ethanol. The degree of inflammation was quantified using a macroscopic damage score (MDS, 0-13 scale) and myeloperoxidase (MPO) activity.

Using the Ussing chamber technique, secretory responses evoked by veratridine (a nerve stimulant, 1x10⁻⁵M) were assessed and expressed as % response to carbachol (1x10-5M).

Longitudinal muscle strips were used for the motility studies and contractile responses to carbachol (1x10-8 - 3x10-5M) were evaluated.

CBD was used at doses of 5, 10, 15 and 20 mg/kg. THC was used at doses of 5, 10 and 20 mg/kg. In addition THC at doses of 5 and 10 mg/kg was administered in combination with CBD at a dose of 10mg/kg.

Doses were administered i.p., once daily starting 0.5 hour before TNBS administration and animals (n=6-11 per group) were sacrificed 72 hours after colitis induction.

### Results:

The use of a TNBS model results in the untreated (control) animals showing a significantly depressed contractile and secretory response, whereas their macroscopic damage score (MDS) and myeloperoxidase (MPO) activity scores are increased.

### CBD alone treatments

Table 2 shows that CBD treatment at 10 mg/kg produced a clear reduction in MDS, but this failed to be statistically significant.

**Table 2: Effects of cannabidiol on macroscopic damage**

| | | Vehicle | CBD 5 | CBD 10 | CBD 15 | CBD 20 |
|---|---|---|---|---|---|---|
| MDS | Mean | 5.46 | 6.75 | 2.83 | 5.60 | 4.60 |
| | SEM | 0.85 | 1.65 | 1.01 | 0.68 | 1.40 |

Table 3 shows that CBD also reduced MPO in a dose dependent manner, but the effect was not significant.

**Table 3: Effects of cannabidiol on myeloperoxidase activity**

| | | Vehicle | CBD 5 | CBD 10 | CBD 15 | CBD 20 |
|---|---|---|---|---|---|---|
| MPO | | 6.03 | 5.38 | 4.65 | 4.00 | 2.68 |
| [ngHRP/mg protein] | Mean | | | | | |
| | SEM | 0.75 | 1.15 | 0.99 | 1.51 | 0.38 |

### THC alone treatments

Table 4 shows that the MDS was lowered by THC at 10mg/kg, the dose-response relationship of this cannabinoid showing a bell-shaped pattern.

**Table 4: Effects of tetrahydrocannabinol on macroscopic damage**

| | | Vehicle | THC 5 | THC 10 | THC 20 |
|---|---|---|---|---|---|
| MDS | Mean | 6.00 | 6.50 | 2.67 | 4.67 |
| | SEM | 0.50 | 0.76 | 1.02 | 0.71 |

Table 5 shows that THC at 10 and 20mg/kg reduced TNBS-induced neutrophil infiltration.

**Table 5: Effects of tetrahydrocannabinol on myeloperoxidase activity**

| | | Vehicle | THC 5 | THC 10 | THC 20 |
|---|---|---|---|---|---|
| MPO [ngHRP/mg protein] | Mean | 3.35 | 4.29 | 1.77 | 2.13 |
| | SEM | 0.38 | 0.44 | 0.12 | 0.23 |

However THC alone did not affect secretory responses to nerve stimulation, in addition responses to carbachol were not significantly increased by THC (data not shown).

### THC and CBD combination treatments

Tables 6 and 7 show that the combined treatment of THC at 5 mg/kg and CBD at 10 mg/kg resulted in reduction of MDS and MPO (4.2±0.5, 2.4±0.3, respectively, P<0.05 v. THC5). Treatment with THC at 10 mg/kg and CBD at 10 mg/kg did not significantly affect these parameters.

**Table 6: Effects of tetrahydrocannabinol in combination with CBD on macroscopic damage**

| | | Vehicle | THC (5mg/kg) + CBD (10mg/kg) | THC (10mg/kg) + CBD (10mg/kg) |
|---|---|---|---|---|
| MDS | Mean | 6.00 | 4.17 | 3.50 |
| | SEM | 0.50 | 0.54 | 0.56 |

**Table 7: Effects of tetrahydrocannabinol in combination with CBD on myeloperoxidase activity**

| | | Vehicle | THC (5mg/kg) + CBD (10mg/kg) | THC (10mg/kg) + CBD (10mg/kg) |
|---|---|---|---|---|
| MPO [ngHRP/mg protein] | Mean | 3.35 | 2.44 | 2.62 |
| | SEM | 0.38 | 0.32 | 0.58 |
| | | | | |

Table 8 shows that the secretory responses to nerve stimulation when treated with THC at 10 mg/kg and CBD at 10 mg/kg were significantly enhanced (62.8±8.8, P<0.05 v. THC10, 30.3±7.5 and vehicle, 24.4±8.2).

**Table 8: Effect of tetrahydrocannabinol in combination with CBD on the secretory response of the mucosa-submucosa preparations to epithelial stimulation**

| | | Vehicle | THC (5mg/kg) + CBD (10mg/kg) | THC (10mg/kg) + CBD (10mg/kg) |
|---|---|---|---|---|
| %response to carbachol | Mean | 25.45 | 35.17 | 62.80 |
| | SEM | 8.17 | 6.11 | 8.02 |

Table 9 shows that the responses to carbachol were significantly increased by THC at 10 mg/kg and CBD at 10 mg/kg were (P<0.05 v. vehicle).

**Table 9: Effects of tetrahydrocannabinol in combination with CBD on the secretory response of the mucosa-submucosa preparations to epithelial stimulation**

| | | Vehicle | THC (5mg/kg) + CBD (10mg/kg) | THC (10mg/kg) + CBD (10mg/kg) |
|---|---|---|---|---|
| Carbachol ΔI_{sc} [µA·cm⁻²] | Mean | 103.8 | 151.3 | 141.6 |
| | SEM | 17.34 | 25.20 | 14.57 |
| | | | | |

In conclusion, THC reduces MPO and tissue injury in the TNBS acute colitis model and CBD potentiates the effects of an ineffective dose of THC on these parameters. Moreover the combination of the two drugs is beneficial in functional studies.

In order to demonstrate more clearly the significance of these data, Table 10 below compares the data generated for CBD at 10 mg/kg; THC at 5 and 10 mg/kg; and the combination of THC at 5 mg/kg and CBD at 10 mg/kg and THC at 10 mg/kg and THC at 10 mg/kg.

**Table 10**

| Test article | MDS | MPO | Contractile response | Secretory response |
|---|---|---|---|---|
| Vehicle | ∼ 6.0 | ∼ 3.3 | ∼ 25 | ∼ 104 |
| CBD (10 mg/kg) | 2.83 | 4.65 | n/a | n/a |
| THC (5 mg/kg) | 6.5 | 4.29 | n/a | n/a |
| THC (10 mg/kg) | 2.67 | 1.77 | n/a | n/a |
| THC (5 mg/kg) and CBD (10 mg/kg) | 4.17 | 2.44 | 35.17 | 151.3 |
| THC (10 mg/kg) and CBD (10 mg/kg) | 3.15 | 2.62 | 62.8 | 141.6 |

Table 10 demonstrates that although a beneficial effect on MDS occurs after treatment with CBD alone the effect on MPO was not significant, meaning that a treatment with CBD alone would only reduce inflammation in relation to macroscopic damage score and as such would not be so effective.

Treatment with THC alone at 10 mg/kg demonstrates a considerable improvement from the control in both MDS and MPO. However a treatment regime where THC was provided at such a high dose may not be suitable for some patients.

The data generated for both combination groups of THC and CBD demonstrate a clear improvement over the control in both inflammatory tests; MDS and MPO. In addition they provide evidence that the contractile and secretory responses are also shown to improve over the control when treated with the combinations.

Therefore a treatment option that provides a combination of THC and CBD may be a better alternative than that of THC alone as it is also known that CBD is able to ameliorate some of the unwanted side effects of THC.

### Example 2 (Reference example):

A double blind, randomised, parallel group, placebo controlled pilot study to investigate the effect of CBD-containing cannabis plant extract in Crohn's disease.

A 13-week (one-week baseline, eight weeks treatment and four-week follow-up), multicentre, double blind, randomised, parallel group, placebo controlled pilot study to evaluate the efficacy and safety of CBD-containing cannabis plant extract in subjects with an exacerbation of Crohn's disease was undertaken. Eligible subjects entered a one-week baseline period. Subjects then returned to the centre for randomisation and dose introduction. Their progress was reviewed after two weeks, four weeks and eight weeks of treatment. Follow-up assessments were done between 7-10 days after week eight by telephone and after a four-week treatment free period, when subjects returned to the centre for final study assessments.

The Primary Objective of the study was to compare the efficacy of CBD-containing cannabis plant extract with placebo using the change from baseline in Crohn's disease activity index (CDAI) score.

The Secondary Objectives of the study were:
- To examine the change from baseline in CDAI score for CBD-containing cannabis plant extract and placebo;
- To compare the efficacy of CBD-containing cannabis plant extract with placebo using the change from baseline in:
   - Harvey Bradshaw Index;
   - C-Reactive Protein (CRP);
   - Erythrocyte Sedimentation Rate (ESR);
   - Platelet count;
   - Circulating interleukin (IL)-2 receptors;
   - IL-6 Inflammatory Bowel Disease Questionnaire (IBDQ); and
   - Neutrophil count.
- To compare for CBD-containing cannabis plant extract with placebo in the percentage of responders and subjects in remission at the end of treatment;
- To evaluate the patient's global impression of change (PGIC) with for CBD-containing cannabis plant extract compared with placebo; and
- To evaluate safety and tolerability of for CBD-containing cannabis plant extract compared with placebo.

### Method:

Eligible subjects had Crohn's disease with active inflammation of the colon and/or terminal ileum and scored between 175 and 450 inclusive on the CDAI. Subjects who had symptoms of intestinal obstruction believed by the investigator to be due to a fibrous stricture or findings suggestive of intestinal perforation were excluded. Subjects who had received TNFα-modifying agents within two months before administration of study drug were also excluded.

The investigational medicinal product (IMP) was presented in a solid dosage format. Each pastille contained 20mg CBD and no more than 1.3mg THC. The maximum permitted dose of IMP was 10 pastilles taken orally within any 24-hour period (200mg CBD). Up to five pastilles were taken in the morning and evening before food with an interval of approximately 12 hours between doses. Subjects followed a stepwise titration to their optimal dose based on efficacy, tolerability and the maximum permitted dose. Placebo, produced to match the taste, smell and appearance of the active formulation but containing no active components, were presented as 1000mg pastilles. Five pastilles were administered orally twice daily prior to food.

Throughout the study, the investigator could prescribe any concomitant medications or treatments deemed necessary to provide adequate supportive care.

The CBD-containing plant extract could be prescribed as an adjunct therapy to existing aminosalicylate, corticosteroid (including inhaled and topical preparations) or immunosuppressive therapies.

Withdrawing existing medication for treatment of Crohn's disease was not considered ethically acceptable, however, any such medication must have remained stable for two weeks (aminosalicylates and corticosteroids) and for four weeks (immunosupressants) prior to IMP administration and for the duration of the study. Subjects could not commence treatment with these medications or nutritional formulas during the study but were allowed to use or initiate treatment with creams, ointments and inhalers containing corticosteroids.

### Crohn's Disease Activity Index (CDAI)

Crohn's disease activity was assessed at Visits 2, 3, 4, 5/withdrawal and 6 using the CDAI. This index comprised eight items providing a global rating score of disease activity calculated from the information recorded in the subject diaries for the seven days prior to each visit and following clinical examination by the investigator at each visit. The number of complications presumed related to Crohn's disease was also scored if present during examination or from symptoms reported by the subject. The determination of the final CDAI score involved the measurement of haematocrit and body weight and the multiplication of each score for the eight items by individual weighting factors. A subject was defined clinically as a 'responder' if they had dropped 70 or more on the CDAI and defined as being in 'clinical remission' if there was a reduction to less than 150 in the CDAI.

### Harvey Bradshaw Index

Disease activity was assessed at Visits 2, 3, 4, 5/withdrawal and 6 using the Harvey Bradshaw Index where subjects rated their general wellbeing, abdominal pain and the number of liquid stools per day for the previous 24 hours. This information was obtained from the subject diary cards.

During each visit, the investigator examined the subject for the presence of an abdominal mass, which was rated on a four-point scale. The complications of Crohn's disease were scored (one per item) if present during examination. Components of the Harvey Bradshaw Index were recorded.

### The Selective Reminding Test (SRT)

The SRT was completed by the subject at baseline (Visit 2) and on completion or withdrawal from the study (Visit 5) as a measure of memory impairment.

### C Reactive Protein (CRP), Erythrocyte Sedimentation Rate (ESR) and Platelet Count

Laboratory markers of inflammation CRP, ESR and platelet count were determined from plasma samples obtained at Visits 2, 3, 4, 5/withdrawal and 6 and analysed by Pivotal (CRP and platelet count only). ESR was analysed by site staff or by a local laboratory, as appropriate.

### Plasma IL-2 receptor and IL-6 levels

Plasma samples were collected from subjects at Visits 2, 4, 5/withdrawal and 6 and sent frozen to Pivotal for analysis of circulating soluble IL-2 receptor and IL-6 levels.

### Inflammatory Bowel Disease Questionnaire (IBDQ)

At Visits 2, 5/withdrawal and 6, subjects completed the IBDQ, a disease specific health-related quality of life questionnaire comprising 32 questions divided into four dimensions as follows: bowel function (10 questions), systemic symptoms (5 questions), social function (5 questions) and emotional function (12 questions).

Each question was graded on a seven-point scale where 1 represents worst function and 7 represents best function. Therefore, the minimum (worst) score was 32 and the maximum (best) score was 224 (70 for the bowel function dimension; 35 for the systemic symptoms dimension; 35 for the social function dimension; 84 for the emotional function dimension). Thus, the higher the score, the better the subject's functional status. The IBDQ has been shown to correlate well with disease activity.

### Patient's Global Impression of Change (PGIC)

The subject gave their impression of the overall change in their condition during the study at Visits 5/withdrawal and 6 using the following scale; 1 = very much improved, 2 = much improved, 3 = minimally improved, 4 = no change, 5 = minimally worse, 6 = much worse, 7 = very much worse.

### Daily Symptom Diary

Subjects were issued with a daily diary to complete throughout the study for assessment of general wellbeing (scored as 0 = generally well, 1 = slightly below par, 2 = poor, 3 = very poor, 4 = terrible), number of bowel actions per day and the proportion of liquid or very soft stools, anti-diarrhoeal use, abdominal pain/cramps (rated as 0 = none, 1 = mild, 2 = moderate, 3 = severe) and oral temperature. The diary was completed at the same time each day.

At Visits 2, 3, 4, 5 /withdrawal and 6, the diary information for the previous seven days (if available at withdrawal) was used to calculate the CDAI score. Subject IMP dosing was recorded in the daily diary.

The following clinical laboratory parameters were also tested:
- Haematology - red blood cells (RBC), haemoglobin, haematocrit, mean cell volume (MCV), platelets and white blood cells (WBC) with automated differential.In the event of a grossly abnormal blood count, a blood film was to be examined.
- Biochemistry - sodium, potassium, urea, creatinine, total bilirubin, alkaline phosphatase, aspartate transferase (AST), alanine aminotransferase (ALT), gamma glutamyl transferase (GGT), albumin, total protein and calcium.
- Urinalysis - the investigator performed routine lab-stix analysis of the following parameters: pH, blood, protein, glucose and nitrites. Pivotal Laboratories performed microscopy on urine samples with abnormal lab-stix results.

### Results:

Of the eight subjects who completed the study, all but one (receiving placebo) reported an improvement in Crohn's disease as determined by the decrease in CDAI score from baseline as shown in Table 11 below.

**Table 11: Summary of Individual CDAI Results**

| Subject | IMP | Total CDAI score - baseline | Total CDAI score - final treatment/ withdrawal | Change in CDAI score (and %) |
|---|---|---|---|---|
| 102 | CBD-CPE* | 197 | 166 | -31 (-16) |
| 107 | CBD-CPE | 244.6 | 46 | -198.6 (-81) |
| 117 | CBD-CPE | 321.5 | 213.54 | -107.96 (-34) |
| 118 | CBD-CPE | 356 | 297.8 | -58.2 (-16) |
| 105 | Placebo | 246.1 | 180.1 | -66 (-27) |
| 106 | Placebo | 203 | 160.9 | -42.1 (-21) |
| 122 | Placebo | 252 | 239.1 | -12.9 (-5) |
| 125 | Placebo | 238 | 330.7 | 92.7 (39) |

| | | | | |
|---|---|---|---|---|
| * CBD-CPE - CBD-containing plant extract | | | | |

The average reduction in CDAI score from baseline in the four completers taking CBD-containing plant extract was 98.94 points or 36.8%, whereas that for placebo was only 28.3 or 3.5%.

This demonstrates a very clear pattern of improvement in the subjects receiving CBD-containing plant extract over those taking placebo.

## Claims

1. A combination of the cannabinoids tetrahydrocannabinol (THC) and cannabidiol (CBD) for use in the treatment of an inflammatory bowel disease, wherein the ratio of THC to CBD (w/w) is 2:1 to 1:2, and wherein the CBD is in a unit dose form suitable for delivering a daily dose of CBD in the dose range of from between 5 to 200mg.

2. A combination of THC and CBD for use according to claim 1, wherein the inflammatory bowel disease is Crohn's disease.

3. A combination of THC and CBD for use according to claim 1, wherein the inflammatory bowel disease is ulcerative colitis.

4. A combination of THC and CBD for use according to any of the preceding claims, wherein the THC and CBD are administered separately, sequentially or simultaneously to one another.

## Patentansprüche

1. Kombination der Cannabinoide Tetrahydrocannabinol (THC) und Cannabidiol (CBD) zur Verwendung in der Behandlung einer entzündlichen Darmerkrankung, wobei das Verhältnis von THC zu CBD (w/w) 2:1 bis 1:2 ist und wobei das CBD in einer Einheitsdosierungsform vorliegt, die geeignet ist, um eine tägliche Dosis von CBD in dem Dosisbereich zwischen 5 und 200 mg zu verabreichen.

2. Kombination von THC und CBD zur Verwendung gemäß Anspruch 1, wobei die entzündliche Darmerkrankung Morbus Crohn ist.

3. Kombination von THC und CBD zur Verwendung gemäß Anspruch 1, wobei die entzündliche Darmerkrankung Colitis ulcerosa ist.

4. Kombination von THC und CBD zur Verwendung gemäß einem der vorangehenden Ansprüche, wobei das THC und CBD getrennt voneinander, nacheinander oder gleichzeitig verabreicht werden.

## Revendications

1. Combinaison des cannabinoïdes tétrahydrocannabinol (THC) et cannabidiol (CBD) pour une utilisation dans le traitement d'une maladie inflammatoire de l'intestin, dans laquelle le rapport de THC sur CBD (p/p) est de 2/1 à 1/2, et dans laquelle le CBD est sous la forme d'une dose unitaire appropriée pour la délivrance d'une dose quotidienne de CBD dans la plage de doses entre 5 et 200 mg.

2. Combinaison de THC et de CBD pour une utilisation selon la revendication 1, où la maladie inflammatoire de l'intestin est la maladie de Crohn.

3. Combinaison de THC et de CBD pour une utilisation selon la revendication 1, où la maladie inflammatoire de l'intestin est la rectocolite hémorragique.

4. Combinaison de THC et de CBD pour une utilisation selon l'une quelconque des revendications précédentes, où le THC et le CBD sont administrés séparément, séquentiellement ou simultanément l'un par rapport à l'autre.
